# EUROPEAN PATENT APPLICATION

(11) **EP 4 717 703 A1**
(43) Date of publication of application: **01.04.2026**
(21) Application number: 24202689.6
(22) Date of filing: 25.09.2024
(51) Int. Cl.: C07K 14/00, C07K 14/705, A61K 38/00, A61P 35/00

(54) **A CEA-BINDING POLYPEPTIDE**

(71) Applicant: Zytox Therapeutics AB, 118 26 Stockholm (SE)
(72) Inventor: NILVEBRANT, Johan, 187 34 Täby (SE); NYGREN, Per-Åke, 178 40 Ekerö (SE); BJÖRNSTEDT, Mikael, 141 39 Huddinge (SE); ACHOUR, Adnane, 113 47 Stockholm (SE); SALTER, Hugh, 191 41 Sollentuna (SE); FERNÁNDEZ MORO, Carlos, 129 56 Hägersten (SE)
(74) Representative: Kransell & Wennborg KB

(57) **Abstract**

The present disclosure generally relates to a CEA-binding polypeptide comprising at least one alpha helix and a CEA-binding motif, wherein the CEA-binding motif is located on the at least one alpha helix and comprises the amino acid sequence XₐX_{b}X_{c}X_{d}Xₑ (SEQ ID NO:1), wherein
Xₐ is F or W;
X_{b} is W;
X_{c}, and X_{d} are individually selected from any amino acid; and
Xₑ is W or Y.

The present disclosure also relates to a fusion protein or conjugate comprising the CEA-binding polypeptide, and to its use in a method of treatment, as well as for vitro diagnosis of the presence and/or level of CEA in a sample.

## Description

### TECHNICAL FIELD

The present disclosure generally relates to a CEA-binding polypeptide comprising at least one alpha helix and a CEA-binding motif, wherein the CEA-binding motif is located on the least one alpha helix. The present disclosure also relates to a fusion protein or conjugate comprising the CEA-binding polypeptide, and to its use in a method of treatment, as well as for in vitro diagnosis of the presence and/or level of CEA in a sample.

### BACKGROUND

Carcinoembryonic antigen (CEA), also known as CEACAM5 (Carcinoembryonic Antigen-Related Cell Adhesion Molecule 5), is a highly glycosylated glycosylphosphatidyl-inositol (GPI)-anchored protein expressed on the surface of cells in the gastrointestinal tract during fetal development. CEA plays an important role in cell adhesion.

Normally, CEA expression essentially stops before birth, but CEA may remain expressed in the GI tract at low levels. However, in certain cancer types, particularly adenocarcinomas, the level of CEA expression is significantly elevated. The overexpression of CEA in tumor tissue renders CEA a valuable diagnostic biomarker as well as a target for directed tumor therapy.

The most common method of detecting CEA in clinical diagnostics involves immunoassays using monoclonal antibodies. Immunohistochemistry (IHC) on tissue samples may be employed to visualize CEA expression in biopsy sections. Enzyme-linked immunosorbent assays (ELISA) may be used to measure CEA levels in blood serum, e.g. to monitor tumor progression or the therapeutic response in cancer patients.

While various monoclonal antibodies (e.g. II-7 and CEA31) have been developed to bind specific epitopes of CEA, the use of antibodies as affinity agents may suffer from cross-reactivity and high background staining, thereby limiting the sensitivity and specificity of CEA detection. Furthermore, monoclonal antibodies are costly diagnostic reagents.

Even though CEA was recognized as a cancer antigen more than 20 years ago, no CEA-targeting monoclonal antibody has yet been approved for therapeutic use, which suggests that monoclonal antibodies may be suboptimal for therapeutic targeting of CEA.

There is consequently a need, both in diagnostics and therapy, for an improved CEA-targeting entity, which shows high specificity and affinity, and which is robust enough to maintain its structural integrity in a variety of biological conditions. Furthermore, such a CEA-targeting entity is preferably readily and reproducibly produced.

### SUMMARY

In view of the above-mentioned problems, it is an object of the present disclosure to provide an improved CEA affinity agent for use in therapeutic and diagnostic applications.

According to a first aspect of the present disclosure, there is provided a CEA-binding polypeptide comprising at least one alpha helix and a CEA-binding motif, wherein the CEA-binding motif is located on the at least one alpha helix and comprises the amino acid sequence XₐX_{b}X_{c}X_{d}Xₑ (SEQ ID NO:1), wherein
Xₐ is For W;
X_{b} is W;
X_{c}, and X_{d} are individually selected from any amino acid; and
Xₑ is W or Y.

The inventors have found that the CEA-binding motif as defined hereinabove enables the polypeptide to selectively bind to CEA with high affinity.

The CEA-binding motif was identified through phage display screening. Phage display screening is a process, in which vast libraries of polypeptides are expressed on the surface of bacteriophages, allowing for a systematic selection of specific binders to a given target, in this case CEA. The use of phage display allows for the exploration of billions of polypeptide variants to identify those with the highest affinity and specificity for CEA.

During the phage display selection, CEA was immobilized, and phages displaying a library of affibody polypeptides, 58 aa in length [1] with potential CEA-binding capabilities were exposed to this target. After several rounds of panning, where non-specific binders were washed away and high-affinity binders were retained, the "top candidates" were isolated. These candidates were further analyzed for binding affinity and specificity.

Through this rigorous selection process, the CEA-binding motif XₐX_{b}X_{c}X_{d}Xₑ (SEQ ID NO:1), as described hereinabove, was identified as a core sequence responsible for binding CEA with high specificity.

Each of the polypeptides representing the "top candidates" contained this CEA-binding motif, and the CEA-binding motif was located on an alpha helix of the polypeptide scaffold.

The inventors identified that the amino acid residues in position Xₐ, X_{b}, and Xₑ are key for binding to CEA.

Accordingly, tryptophan (W) or phenylalanine (F) in position Xₐ, tryptophan (W) in position X_{b}, and tryptophan (W) or tyrosine (Y) in position Xₑ are responsible for binding to CEA.

The importance of these specific residues in the CEA-binding motif was further demonstrated in a series of experiments described in the Example section. During these experiments, site-directed mutagenesis was performed to systematically replace the tryptophan (W) residues with an alternative amino acid (alanine) in a CEA-binding polypeptide containing tryptophan in position Xₐ, X_{b}, and Xₑ. It was observed that the binding ability to CEA was significantly reduced or completely lost when the tryptophan residues were substituted with alanine. These findings confirm that the residues in positions Xₐ, X_{b}, and Xₑ are important for the high-affinity binding of the polypeptide to CEA.

Another advantage of the CEA-binding polypeptide of the present disclosure is its small size. Since the CEA-binding polypeptide is significantly smaller than an antibody, it allows for deeper tissue penetration. This is important in both therapeutic and diagnostic applications (e.g. immunohistochemistry (IHC)). Furthermore, the polypeptide may be produced recombinantly in bacteria, thereby avoiding the complexities and high costs associated with monoclonal antibody production in mammalian cells.

Moreover, the size of the CEA-binding polypeptide also enables production by chemical peptide synthesis, further simplifying the process, and enables introduction of non-natural groups for e.g. site-directed conjugation.

In exemplary embodiments, X_{c} may be H, Y, D, V, F, K, G, N, E, or A.

In exemplary embodiments, X_{d} may be A or F.

Although the amino acid residues at these positions (X_{c} and X_{d}) are not directly involved in the binding interaction with CEA, they may contribute to an enhanced structural stability of the polypeptide. These residues may e.g. play a role in maintaining optimal spatial arrangement of the binding surface, which may indirectly improve the polypeptide's ability to form a more stable complex with CEA.

In exemplary embodiments, the CEA-binding motif may comprise the amino acid sequence as defined in any one of SEQ ID NO:2-14, or a corresponding sequence having at least 80% sequence identity with any one of SEQ ID NO:2-14.

As used herein, the term "at least 80% sequence identity" refers to a comparison between two amino acid sequences, where 80% or more of the residues in one sequence are identical to the corresponding residues in the other sequence when aligned. Sequence identity is typically measured over the full length of the sequences being compared, in this case across the length of the CEA-binding motif.

In practice, this means that a sequence having at least 80% sequence identity with any one of SEQ ID NO:2-14 may differ in up to 20% of the amino acids, provided that the core residues essential for the functionality of the CEA-binding motif are maintained.

The CEA-binding motifs defined by SEQ ID NO:2-14 are illustrated in table 1 hereinbelow.

**Table 1: CEA-binding motifs according to SEQ ID NO:2-14**

| SEQ ID NO | Amino acid sequence |
|---|---|
| 2 | WWFAW |
| 3 | WWKAY |
| 4 | WWHAY |
| 5 | WWYAY |
| 6 | WWDAY |
| 7 | FWVAY |
| 8 | FWFAY |
| 9 | WWAAW |
| 10 | WWGAY |
| 11 | FWNAW |
| 12 | FWEAY |
| 13 | WWAFY |
| 14 | FWAFY |

The CEA-binding motifs defined by SEQ ID NO:2-14 were identified across all 19 of the top candidates from the phage display process. The key residues tryptophan (W), phenylalanine (F) and tyrosine (Y), are highly conserved in these binding motifs, suggesting an important localized hydrophobic interaction with CEA.

In exemplary embodiments, the CEA-binding polypeptide may have a three-helix bundle structure and may comprise a first alpha helix, a second alpha helix, and a third alpha helix; the second alpha helix being arranged C-terminally of the first alpha helix; the third alpha helix being arranged C-terminally of the second alpha helix.

As used herein, the term "three-helix bundle structure" refers to a polypeptide scaffold comprising three alpha helices that are arranged in a compact, folded configuration. The helices are typically packed closely together in parallel or antiparallel orientations, stabilized by hydrophobic core interactions and other non-covalent bonds. The three-helix bundle anti-parallel conformation provides a framework that supports the display of the CEA-binding motif in a structurally favorable orientation. This conformation helps maintaining the structural integrity of the polypeptide and allows for high-affinity interactions with CEA.

The present disclosure is, however, not limited to a three-helix bundle structure. It is also conceivable that the CEA-binding polypeptide comprising the CEA-binding motif as defined by SEQ ID NO: 1-14, may be introduced into or form part of alternative scaffolds. These may include other alpha-helical configurations, such as single or double alpha helices, or scaffolds comprising mixed alpha/beta structures.

Preferably, the CEA-binding polypeptide has a three-helix bundle structure.

The CEA-binding polypeptide may e.g. comprise the following structure: [N-terminal portion]-[first alpha helix]-[first separating portion]-[second alpha helix]-[second separating portion]-[third alpha helix]-[C-terminal portion].

Preferably, the CEA-binding motif is located on the first alpha helix or the second alpha helix of the three-helix bundle structure.

This allows for the key binding interactions to be maintained in a stable and functional configuration.

Through the vast phage display screening and subsequent mutational analysis performed by the inventors, two different "categories" of CEA-binding polypeptides were identified. In the first category, the CEA-binding motif is located on the first alpha helix. In the second category, the CEA-binding motif is located on the second alpha helix.

Accordingly, in exemplary embodiments, the CEA-binding motif may be located on the first alpha helix of the three-helix bundle structure and may comprise the amino acid sequence defined by any one of SEQ ID NO:2-12, or a corresponding sequence having at least 80% sequence identity with any one of SEQ ID NO:2-12.

The inventors identified that these sequences, when located on the first alpha helix, provided an optimal scaffold for CEA binding.

In alternative embodiments, the CEA-binding motif may be located on the second alpha helix and may comprise the amino acid sequence defined by SEQ ID NO: 13 or SEQ ID NO: 14, or a corresponding sequence having at least 80% sequence identity with SEQ ID NO: 13 or SEQ ID NO: 14.

The sequences defined by SEQ ID NO: 13 and SEQ ID NO: 14 were identified through similar phage display selections, and showed high binding efficiency when located on the second alpha helix. The second alpha helix may be associated with a different spatial arrangement and orientation, which allows for the CEA-binding motif to engage with CEA through an alternative set of interactions. The difference in the positioning of the binding motif between the two categories allows for versatility in the design of the CEA-binding polypeptides.

This "dual-category approach" enables the development of affinity agents that can be adapted for a variety of diagnostic or therapeutic uses, depending on the configuration of the three-helix bundle and the spatial arrangement of the CEA-binding motif.

In exemplary embodiments, the CEA-binding polypeptide may be derived from a domain of *Staphylococcus aureus* Protein A.

*Staphylococcus aureus* Protein A is a well-known bacterial protein, which constituting domains may be used as a structural basis or "scaffold" in protein engineering.

The domain of *Staphylococcus aureus* Protein A is typically a mutated domain. Typically, the domain is a mutated form of the B domain of Protein A, denoted domain Z.

This domain was initially constructed to improve the stability towards certain treatments. Further work has led to variants of the original Z domain having replacements in the first part of the protein, in the loop connecting the first two alpha helices and in the exposed surface of the third helix, distant from the binding surface. Accordingly, domain Z has a large tolerance for amino acid replacements despite its small size of only 58 amino acids.

Domain Z provides a stable scaffold that can be adapted for various target interactions by replacement of its natural binding affinity for the Fc region of antibodies, by e.g. CEA-binding. The inherent stability and refolding ability of domain Z also constitutes a potential for the development of polypeptides remaining intact or retaining their structural integrity after various biological and chemical conditions, e.g. high temperatures, and extreme pH.

Furthermore, domain Z is a small protein (~7 kDa), which enables improved tissue penetration, especially in dense tumor tissues compared to e.g. antibodies. The small non-binding surface area also minimizes non-specific binding, which may be a problem with larger proteins. Novel target binding polypeptides derived from domain Z do not naturally bind to immunoglobulins (IgG), since the amino acids in the original IgG-binding surface have been replaced. The absence of native immunoglobulin binding activity eliminates potential cross-reactivity with antibodies. Hence, Domain Z is an ideal scaffold for development of target binding polypeptides without unwanted interactions.

The CEA-binding polypeptide may be defined by: [first alpha helix]-[separating portion]-[second alpha helix].

The CEA-binding motif is located on either the first alpha helix or the second alpha helix. The separating portion may comprise a loop portion.

The CEA-binding polypeptide may comprise the amino acid sequence: XₐX_{b}X_{c}X_{d}XₑX₁₄X₁₅X₁₆X₁₇X₁₈X₁₉X₂₀X₂₁X₂₂X₂₃X₂₄X₂₅X₂₆X₂₇X₂₈X₂₉X₃₀X₃₁X₃₂X₃₃X₃₄X₃₅ (SEQ ID NO:53), wherein
X₁₄ is H, S, or A, preferably S, or A;
X₁₅ is E,
X₁₆ is I;
X₁₇ is H, R, V, I, M, or K, preferably V, or I;
X₁₈ is D, S, I, E, N, T, V, or H, preferably N, or T;
X₁₉ is L;
X₂₀ is P;
X₂₁ is N;
X₂₂ is L, G, or A;
X₂₃ is N, or T;
X₂₄ is I, D, S, T, K, R, A, V, E, M, or Q, preferably K, or R;
X₂₅ is K, R, E, T, D, N, I, G, A, Q, or F, preferably D, or N;
X₂₆ is Q;
X₂₇ is R, H, T, Q, S, E, I, L, V, or N, preferably Q, or S;
X₂₈ is K, H, V, R, S, G, T, M, Q, D, or I, preferably V, or S;
X₂₉ is A;
X₃₀ is F;
X₃₁ is I or K;
X₃₂ is F, A, K, R, H, Q, or L, preferably H, or R;
X₃₃ is S;
X₃₄ is L; and wherein
X₃₅ is A, V, I, L, S, T, or M, preferably V.

In the CEA-binding polypeptide comprising the amino acid sequence SEQ ID NO:53, the CEA-binding motif is located on the first alpha helix. Xₐ-X₁₈ typically correspond to the amino acid residues of the first alpha helix. X₁₉-X₂₃ represent amino acid residues located in the separating portion, i.e. the loop portion. X₂₄-X₃₅ typically correspond to the amino acid residues of the second alpha helix.

While the amino acid residues in position Xₐ, X_{b}, and Xₑ of the CEA-binding motif are directly involved in the binding with CEA, the amino acid residues in the remaining positions in SEQ ID NO:53 may still contribute to several structural and functional advantages that enhance the overall performance of the CEA-binding polypeptide.

For example, an enhanced structural stability of the polypeptide may be provided. Furthermore, these residues may aid in maintaining an optimal orientation of the binding motif. For example, the polypeptide may adopt a tight and compact fold, and reduce potential steric hindrance at the binding interface. Furthermore, the polypeptide defined hereinbefore may contribute to a higher binding specificity.

The inventors have found that the amino acid residue X₃₅ may also contribute to an improved binding to CEA. Preferably, X₃₅ is V.

The amino acid residues in the [separating portion] may vary, and generally has little impact on the CEA-binding capacity of the CEA-binding polypeptide.

Alternatively, the CEA-binding polypeptide may comprise the amino acid sequence:
X₉X₁₀X₁₁X₁₂X₁₃X₁₄X₁₅X₁₆X₁₇X₁₈X₁₉X₂₀X₂₁X₂₂X₂₃X₂₄X₂₅X₂₆XₐX_{b}X_{c}X_{d}XₑX₃₂X₃₃X₃₄X₃₅ (SEQ ID NO:54), wherein
X₉ is Q, or M;
X₁₀ is V, or Q;
X₁₁ is M, or A;
X₁₂ is A;
X₁₃ is I, or T;
X₁₄ is K, or R;
X₁₅ is E,
X₁₆ is I;
X₁₇ is I, or M;
X₁₈ is K, or S;
X₁₉ is L;
X₂₀ is P;
X₂₁ is N;
X₂₂ is L, G, or A;
X₂₃ is N, or T;
X₂₄ is A, or G;
X₂₅ is N, or Q;
X₂₆ is Q;
X₃₂ is H;
X₃₃ is S;
X₃₄ is L; and
X₃₅ is R, or Y.

In the CEA-binding polypeptide comprising the amino acid sequence SEQ ID NO:54, the CEA-binding motif is located on the second alpha helix. X₉-X₁₈ typically correspond to the amino acid residues of the first alpha helix. X₁₉-X₂₃ represent amino acid residues of the separating portion, i.e. the loop portion. X₂₄-X₃₅ typically correspond to the amino acid residues of the second alpha helix.

As mentioned hereinbefore, the CEA-binding polypeptide may comprise the following structure: [N-terminal portion]-[first alpha helix]-[first separating portion]-[second alpha helix]-[second separating portion]-[third alpha helix]-[C-terminal portion].

The first alpha helix may comprise the amino acids X₉-X₁₈ in the sequences described hereinbefore.

The second alpha helix may comprise the amino acids X₂₄-X₃₅ in the sequences described hereinbefore.

The third alpha helix may comprise an amino acid sequence as defined by ANLLAEAKKLNDA (SEQ ID NO:55);
SELLSEAKKLNDS (SEQ ID NO:56); or
a corresponding sequence having at least 70% sequence identity with SEQ ID NO:55 or SEQ ID NO:56.

In exemplary embodiments, the CEA-binding polypeptide may comprise the amino acid sequence of any one of SEQ ID NO: 15-SEQ ID NO:33 or a corresponding sequence having at least 70% sequence identity with any one of SEQ ID NO: 15- SEQ ID NO:33.

The sequences are listed in table 2 hereinbelow, along with their affibody reference used in the Example section.

**Table 2: CEA-binding polypeptides as defined by SEQ ID NO:15-33**

| SEQ ID NO: | Affibody ref. | |
|---|---|---|
| 15 | A8 | WWHAYHEIHDLPNLNIKQRKAFIFSLM |
| 16 | A9 | WWYAYSEIHSLPNLNDRQHHAFIASLI |
| 17 | A12 | WWDAYHEIRILPNLNSEQTVAFIKSLM |
| 18 | C7 | FWVAYHEIRELPNLNTTQTRAFIRSLS |
| 19 | C9 | WWFAWSEIVNLPNLNKDQQVAFKHSLV |
| 20 | C11 | WWKAYAEIITLPNLNRNQSSAFIRSLV |
| 21 | C12 | WWHAYHEIMTLPNLNRIQQHAFIQSLM |
| 22 | D8 | WWKAYHEIRDLPNLNAEQRGAFIQSLM |
| 23 | D10 | WWKAYHEIRTLPNLNVEQETAFIRSLA |
| 24 | D12 | FWFAYHEIRVLPNLNAGQSHAFIQSLT |
| 25 | E9 | WWHAYHEIHDLPNLNDAQIMAFKKSLM |
| 26 | E11 | WWHAYHEIKDLPNLNEGQLSAFIRSLM |
| 27 | F7 | WWGAYHEIRELPNLNDEQVHAFIRSLM |
| 28 | G9 | WWAAWSEIMVLPNLNMQQRQAFKQSLV |
| 29 | G10 | FWNAWHEIRVLPNLNQFQTDAFILSLM |
| 30 | G12 | FWEAYHEIITLPNLNKEQNIAFIRSLL |
| 31 | H12 | WWYAYHEIIHLPNLNIGQTKAFIRSLV |
| 32 | B6 | QVMAIKEIIKLPNLNANQWWAFYHSLY |
| 33 | H11 | MQAATREIMSLPNLNGQQFWAFYHSLR |

In exemplary embodiments, the CEA-binding polypeptide may be defined by the amino acid sequence of any one of SEQ ID NO:34-SEQ ID NO:52 or a corresponding sequence having at least 70% sequence identity with any one of SEQ ID NO:34-SEQ ID NO:52.

According to another aspect, there is provided a fusion protein or conjugate comprising at least:
- a first part comprising the CEA-binding polypeptide as described hereinbefore; and
- a second part comprising at least one additional CEA-binding polypeptide as described hereinbefore, a therapeutic agent, a labelling agent, a half-life-extending region, and/or a second target-binding polypeptide.

The fusion protein may be a dimeric fusion of two CEA-binding polypeptides.

Alternatively, the fusion protein or conjugate may have trimeric or any other multimeric configuration. A dimer or a multimer of the CEA-binding polypeptide may enhance the overall binding strength through avidity effects (see Example section).

In therapeutic applications, dimeric or multimeric CEA-binding polypeptides may enhance the target efficiency by an enhanced binding strength to CEA-expressing tumor cells, and/or enhance internalization. This may be useful when delivering therapeutic agents, such as cytotoxic drugs or radionuclides, ensuring that the fusion protein remains attached to the cancer cells, enhancing the effect of the therapeutic agent. In diagnostic applications, fusion proteins comprising dimers or other multimers may provide stronger binding and amplified signal, which may lead to a clearer visualization of tumor cells.

In exemplary embodiments, the first part may comprise the CEA-binding polypeptide defined in SEQ ID NO:2, SEQ ID NO:3, SEQ ID NO: 19, or SEQ ID NO:20 and wherein the second part may comprise at least one additional CEA-binding polypeptide as defined in SEQ ID NO:2, SEQ ID NO:3, SEQ ID NO: 19, or SEQ ID NO:20.

In one preferred embodiment, the fusion protein or conjugate is a homo-dimer comprising a sequence as defined in SEQ ID NO:2, SEQ ID NO:3, SEQ ID NO: 19, or SEQ ID NO:20.

As clearly demonstrated in the Example section, homodimers of the C9-C9 or C11-C11 polypeptides are associated with a significantly enhanced binding to CEA in tissue sections. The C9-C9 dimer performed surprisingly well in immunohistochemical (IHC) studies by producing a high-definition staining of CEA with minimal background noise. In a clinical pathology setting, this may lead to more precise identification of cancerous tissues. Furthermore, the C9-C9 dimer showed a robust performance in formalin-fixed, paraffin-embedded (FFPE) tissues, which indicates that the dimeric construct can maintain strong and specific binding even when subject to challenging or stringent processing steps.

Alternatively, or additionally, the second part may comprise a labelling agent. The labelling agent may be a fluorophore, chromophore, radioactive isotope, biotin (or analogue), hapten, or an enzyme.

Accordingly, the fusion protein or conjugate may be used in diagnostic applications, e.g. for detection and visualization of CEA-expressing tumors.

When conjugated with a fluorophore, the fusion protein enables fluorescence-based detection of CEA in tissue samples. This may be useful for fluorescence microscopy or fluorescent imaging techniques.

A chromophore allows for colorimetric detection, which is commonly used in immunohistochemistry (IHC). The chromophore is deposited at the binding site, and a following enzymatic conversion generates a visible color change, thereby facilitating visually detecting the presence of CEA in tumor samples.

A radioactive isotope may be useful for in vivo diagnostic imaging, e.g. by means of positron emission tomography (PET) or single-photon emission computed tomography (SPECT), where the radioactive signal indicates CEA-expressing tumors.

The conjugation with an enzyme may be useful in enzyme-linked assays.

It is also conceivable that the labelling agent is coupled through a hapten, a biotin or a biotin analogue, which is attached to the CEA-binding polypeptide.

Alternatively, or additionally, the second part may comprise a therapeutic agent, wherein the therapeutic agent is a cytotoxic agent or a radionuclide.

Combining the CEA-binding polypeptide(s) with a cytotoxic agent or a radionuclide allows for the fusion protein or conjugate to function as a targeted therapy for treating CEA-expressing cancers. The cytotoxic agent or radionuclide may be delivered directly to the tumor site, where it can exert its therapeutic effect while minimizing harm to healthy tissues.

Therapeutic radionuclides may be coupled to the polypeptide by different chemistries depending on the nature of the radionuclide, e.g. iodine-131 is attached in a chemical reaction whereas radioactive metal ions like Ac-223 and Lu-177 are chelated into a chelating moiety, previously attached to the targeting moiety by for example maleimide coupling to a unique cysteine, introduced for this purpose.

The cytotoxic agent is configured to kill and/or inhibit the growth of cancer cells after being released inside or in their vicinity. The cytotoxic agent may be a chemotherapeutic agent.

The cytotoxic agent may be configured to directly or indirectly trigger programmed cell death (apoptosis) in cancer cells, which helps in reducing the tumor size and spread. For example, the cytotoxic agent may cause direct damage to the DNA of cancer cells, leading to cell death by apoptosis or ferroptosis. The cytotoxic agent may also be configured to disrupt the processes required for cell division and thereby prevent cancer cells from proliferating.

The present disclosure is not limited to a specific cytotoxic agent, but any cytotoxic agent known to the skilled person may be used. For example, the cytotoxic agent may be a cytotoxic molecule, peptide, or a protein.

The cytotoxic molecule may e.g. be Doxorubicin (DOX), Duocarmycins (DUO), Docetaxel (DTX), Monomethyl auristatin E (MMAE), Monomethyl auristatin F (MMAF), Paclitaxel (PTX), Mertansine (DM1), emtansine (DM1), Ravtansine (DM4), Soravtansine (DM4), Deruxtecan (DXd), PPyrrolobenzodiazepine (PBD), Calicheamicin, or govitecan (SN38).

Examples of cytotoxic proteins are Pseudomonas exotoxin (PE) and engineered variants thereof, including PE38, and diphtheria toxin (DT).

Examples of proteins recruiting cytotoxicity directly or indirectly are targeting domains against immunomodulatory targets such as CD3, CD47, PD-1, PD-L1, CTLA-4, 4-1BB, LAG-3 (Lymphocyte-Activation Gene 3), TIGIT (T-Cell Immunoreceptor with Ig and ITIM Domains), TIM-3 (T-cell Immunoglobulin and Mucin-3), VISTA (V-domain Ig Suppressor of T Cell Activation), B7-H3 (CD276), IDO (Indoleamine 2,3-dioxygenase), B7-H4, BTLA (B and T Lymphocyte Attenuator), KIRs (Killer-Cell Immunoglobulin-Like Receptors), NKG2A (Natural Killer Group 2A), CD73, A2AR (Adenosine A2A Receptor), GITR (lucocorticoid-Induced Tumor Necrosis Factor Receptor).

When the therapeutic agent is a radionuclide, targeted radiation therapy may be delivered to cancerous cells expressing CEA.

Examples of therapeutic radionuclides are ¹⁷⁷Lu, ⁹⁰Y, ¹⁸⁸Re; ¹⁸⁶Re;¹⁶⁶Ho, ¹⁵³Sm, ⁶⁷Cu , ⁶⁴Cu, ¹⁴⁹Tb, ¹⁶¹Tb, ⁴⁷Sc; ²²⁵Ac; ²¹²Pb; ²¹³Bi, ²¹²Bi, ²²⁷Th, ²²³Ra; ^{58m}Co, ¹³¹I, ⁷⁶As, ⁷⁷As and ²¹¹At.

Alternatively, the fusion protein or conjugate on its own or in combinations as outlined hereinbefore may be used for targeting therapeutic particles of various types, including viruses such as oncolytic viruses, or engineered cancer killing cells such as CAR-T cells.

As mentioned hereinbefore, the fusion protein or conjugate may comprise a half-life extending domain.

Any half-life extending domain known to the skilled person may be used. Preferably, the half-life-extending domain is a mutated version of the albumin-binding domain (ABD) from *Streptococcal* protein G.

ABD binds to serum albumin, a naturally abundant protein with a long half-life. By binding to albumin, the fusion protein "piggybacks" on the long half-life of albumin, and thereby obtains an extended circulation time.

Alternatively, or additionally, the second part may comprise a second target-binding polypeptide. The second target-binding polypeptide may target a protein which is different from CEA. For example, the second target-binding polypeptide may target a different oncoprotein. Accordingly, a "dual targeting" fusion protein or conjugate is provided. The dual targeting fusion protein or conjugate may have an enhanced selectivity for a target cell, for example by combined binding of two oncoproteins. Alternatively, the second target-binding polypeptide may target proteins situated on a second cell type, comprising an immune cell.

According to another aspect, there is provided a conjugate comprising at least:
- a first part comprising the CEA-binding polypeptide or the fusion protein or conjugate as described hereinbefore; and
- a nanoparticle.

The nanoparticle preferably comprises a therapeutic agent, e.g. an mRNA or a cytotoxin, typically by encapsulating it. The nanoparticle may facilitate targeted delivery and/or controlled release of the therapeutic agent.

The first and the second parts may be linked covalently, directly or via a linker, and together provide tissue-tropic delivery of the nanoparticle to cells expressing CEA.

According to another aspect, there is provided the CEA-binding polypeptide, fusion protein or conjugate as described hereinbefore for use in a method of treatment of a disorder.

The disorder may be any disorder associated with an overexpression of CEA. For example, the disorder may be cancer or another hyperproliferative disease. Typically, the disorder is cancer.

The cancer may be an adenocarcinoma, e.g. colon cancer, gastric, pancreatic, breast, and lung cancer.

These cancers are associated with an overexpression of CEA.

The CEA-binding polypeptide, fusion protein or conjugate may be combined with a pharmaceutically acceptable carrier. A composition comprising the CEA-binding polypeptide, fusion protein or conjugate, and the pharmaceutically acceptable carrier may e.g. be adapted for intravenous, intraperitoneal, intrathecal, or subcutaneous injection.

According to another aspect there is provided the use of the CEA-binding polypeptide, or the fusion protein or conjugate as described hereinbefore for the in vitro diagnosis of the presence and/or level of CEA in a sample.

For example, the in vitro diagnostic method may be immunohistochemistry (IHC).

According to another aspect, there is provided the use of the CEA-binding polypeptide as described hereinbefore, or the fusion protein or conjugate as described hereinbefore for determining the presence, distribution and/or level of CEA in a sample by immunohistochemistry.

The distribution of CEA may for example be the sub-cellular localization of CEA.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings.

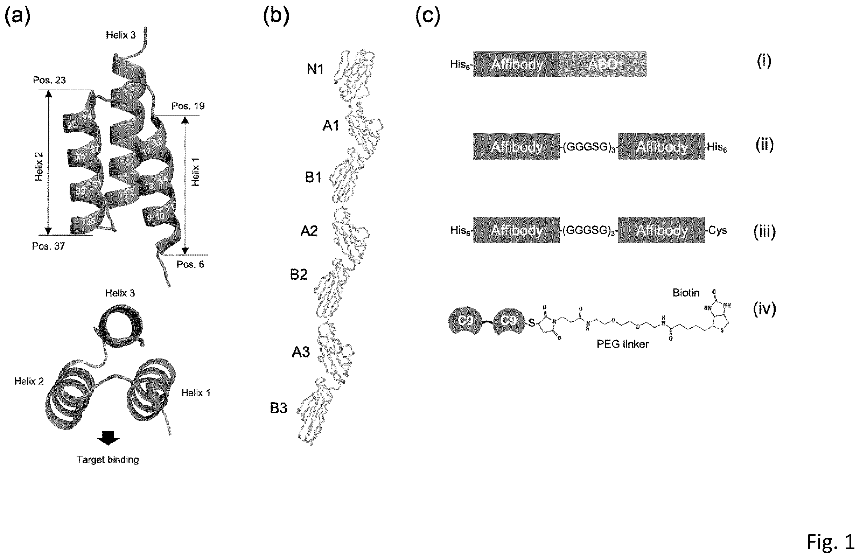

Figure 1: Schematic description of the main proteins included in this study, (a) Overall three-dimensional structure of the 58 aa affibody scaffold, showing the three-helix bundle organization. The 14 positions in helices 1 and 2 that are subjected to combinatorial randomization to construct libraries are highlighted and numbered. (b) Molecular model of the seven-domain human CEACAM5 3D-structure 1E07.pdb previously validated using solution scattering data [3]. (c) Schemes of the different affibody formats used within the present study including (i) His₆-affibody-ABD, where ABD is a 46 aa serum albumin binding domain, (ii) affibody-affibody-His₆, (iii) affibody-His₆, (iv) His₆-affibody-affibody-Cys and a dimeric anti-CEA affibody construct conjugated to maleimide-PEG-biotin (C9-C9-Bio).

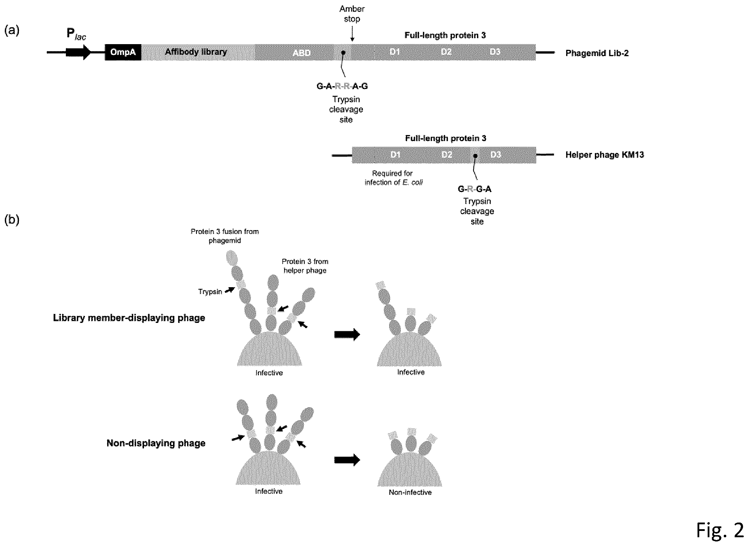

Figure 2: Schematic description of the phagemid and helper phage used in Lib-2. (a) Block diagrams of elements in the phagemid library member expression cassette and the protein 3 encoded by the KM13 helper phage, respectively. The phagemid expression cassette contains several elements including i) a signal peptide from the *E. coli* OmpA protein, (ii) an affibody library member, (iii) the albumin binding domain (ABD), (iv) the trypsin-sensitive sequence, defined by SEQ ID NO:57 (GARRAG), (v) a suppressible amber (TAG) stop codon, and (vi) the full-length M13 phage protein 3. The KM13 helper phage encodes the engineered full-length protein 3, into which the trypsin-sensitive sequence, defined by SEQ ID NO: 58 (GRGA) has been introduced between domains D2 and D3 of the protein. (b) During phage particle assembly in the *E. coli* periplasm, protein 3 molecules encoded from both the phagemid (including the affibody and ABD elements) and the helper phage, compete with each other. The system is intentionally designed for a preferential incorporation of protein 3 from the helper phage, resulting in a large fraction of non-affibody-displaying phage particles. However, in the fraction of phages that actually display affibody-ABD-protein 3 fusion proteins, the display is monovalent (one fusion protein per phage particle), facilitating affinity selections between affibodies and target proteins on a 1:1 interaction basis, avoiding avidity effects from cooperative binding events that could otherwise contribute to biases in the selection pressure. The introduction of trypsin-sensitive sites in both the phagemid and the helper phage enables both trypsin-mediated elution of target-binding phage particles, typically from target-containing beads, and a simultaneous elimination of unwanted non-affibody displaying phage particles during selections. Whereas affibody-ABD fusion displaying phage particles after trypsin cleavage still carries a full-length protein 3 protein that can mediate *E. coli* infection, necessary for the preparation of enriched phages for next selection cycle, non-affibody-ABD fusion displaying phage particles will only display a truncated protein 3 (only domain 3) after trypsin cleavage, that is unable to mediate infection. It should also be noted that during phage display selections, the combined use of phagemids and helper phage results in the production of four types of phage particles, of which only two are presented here.

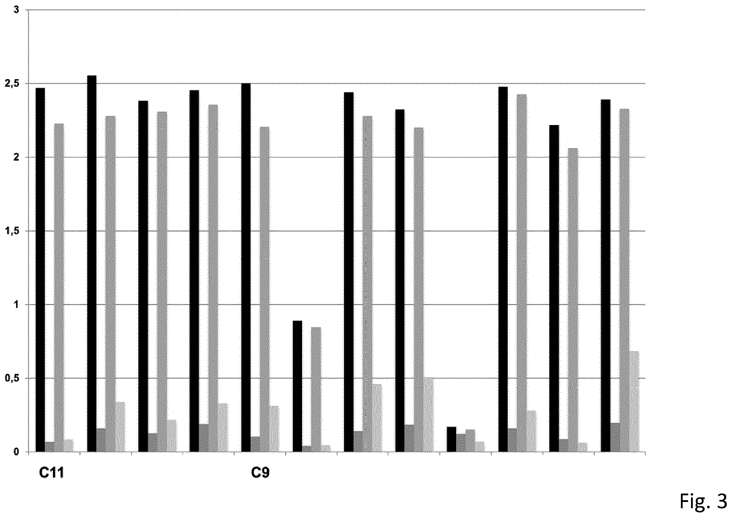

Figure 3: Initial analyses of target specificity using monoclonal phage ELISA. Representative data from phage-ELISA analyses of individual phage stock preparations of clones after the fourth round of selection. Phage particles were assessed for their binding capacity to four ligands: (i) HSA (positive control, black bars), (ii) streptavidin (negative control, dark gray bars), (iii) CEACAM5/hCEA (target, gray bars) and (iv) BSA (negative control, light gray bars). Clones C11 and C9 are highlighted.

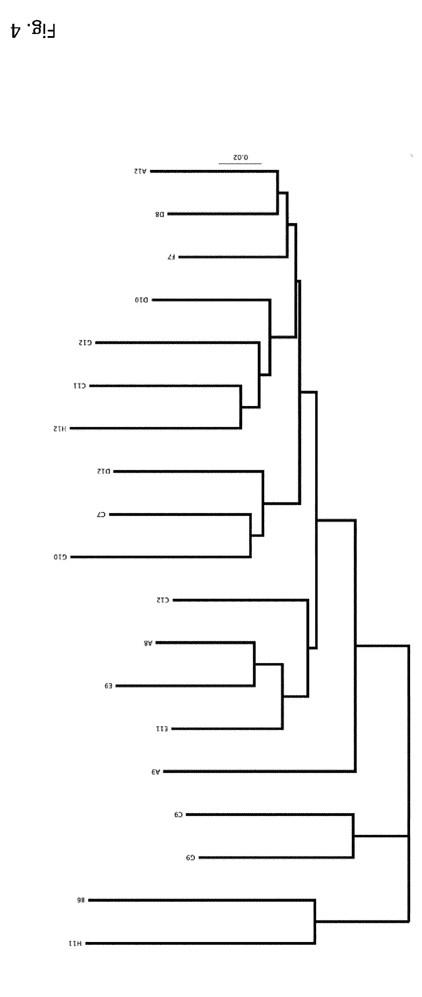

Figure 4: Phylogenetic analysis of the 19 unique phage-ELISA positive clones. Interestingly, a large ensemble of different clusters contained the same sequence several times (from 2x to 24x), indicating an enrichment during the selection rounds. One representative clone per cluster (indicated by arrows) was used to assign all 19 unique clones with a name (e.g., H11 for the cluster at the top).

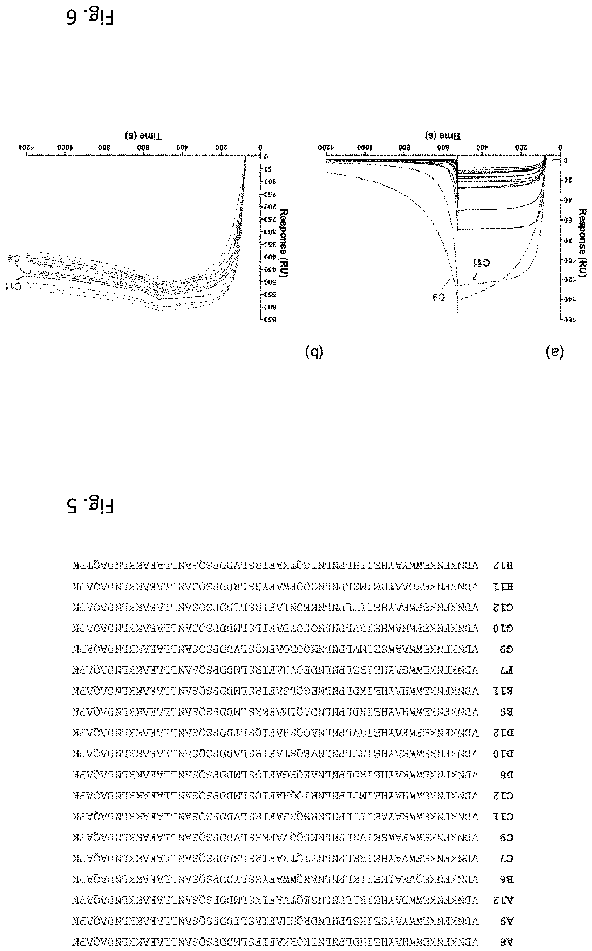

Figure 5: Amino acid sequences of the 19 identified unique affibody clones. An alignment of 19 unique clones reveals the clustering of aromatic residues on either the first or the second helix.
Figure 6: Biosensor binding analyses of the 19 unique anti-CEA affibody clones. Individual His₆-affibody-ABD fusion proteins corresponding to the 19 unique clones were injected at a concentration of 200 nM over sensor chip surfaces containing either hCEA (a) or HAS (b). The results from the hCEA sensor chip surface demonstrate that all 19 clones bind to hCEA, but with different characteristics. Clones C9 and C11 displayed the strongest binding responses, and the slowest off-rate kinetics. The results from injections of the same 19 samples over the HAS sensor chip surface confirmed that all variants were injected at approximately similar concentrations, and that none of these variants formed aggregates.

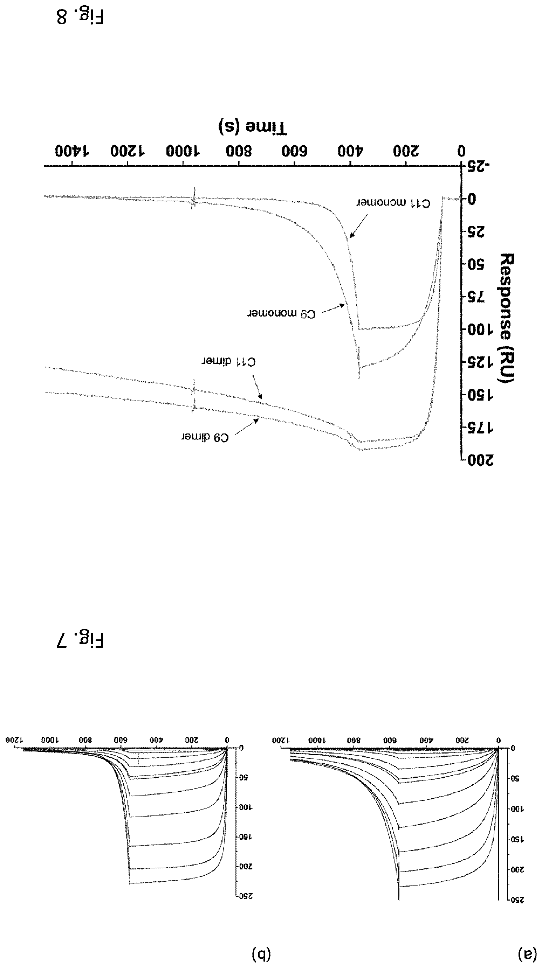

Figure 7: SPR analysis of C9 C11 binding kinetics. A series of concentrations of C9-ABD (a) or C11-ABD (b) fusion proteins ranging between 1-2000 nM were injected over a sensor chip surface containing immobilized hCEA. Using BIAevaluation software for data fitting, the affinities (K_{D}) for both fusion proteins could be determined to ca. 200 nM.
Figure 8: Homodimeric affibody constructs bind with significantly higher affinity to CEA compared to monomers. Proteins were injected at 200 nM concentrations over a sensor chip surface containing immobilized human CEA protein. (a) Binding of monomeric C9-His₆ and C11-His₆ proteins, and (b) binding of homodimeric His₆-C9-C9-ABD and His₆-C11-C11-ABD proteins to hCEA. Note the significantly slower off-rate kinetics for homodimeric affibody constructs, via avidity effects.

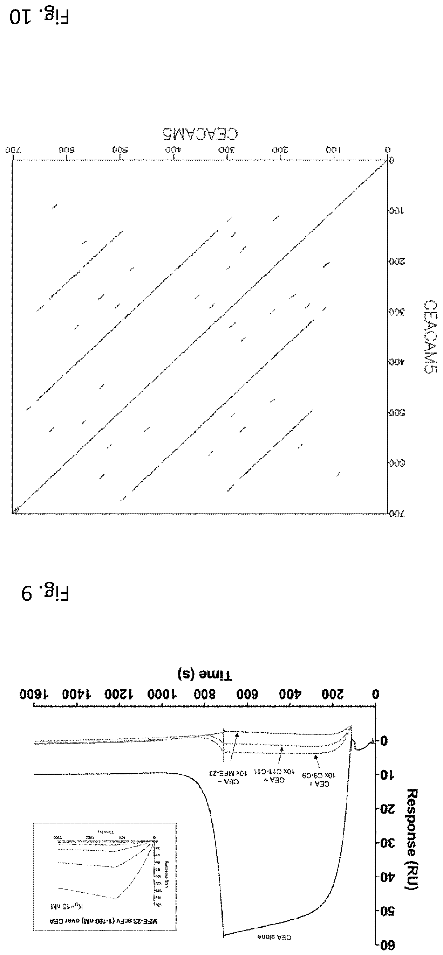

Figure 9: Biosensor binding competition experiments reveal the high specificity of dimeric affibody constructs to hCEA. Binding of either free hCEA or hCEA mixed with a 10-fold molar excess of either C9-C9-His₆, C9-C9-His₆ or anti-hCEA MFE-23 single chain Fy (scFv) proteins [2] to a sensor chip surface containing immobilized C9-C9-His₆, demonstrated that all three analytes blocked binding of hCEA to C9-C9-His₆. Inset: Analysis of the binding of a concentration series (100-1 nM, 3-fold dilutions) of free anti-hCEA MFE-23 scFv to a sensor chip surface containing immobilized hCEA.
Figure 10: Analysis of repeat sequences in hCEA. The analysis was performed using the Dotmatcher software [4] and shows how the A and B domains (residues 145-675), but the not N-terminal N1 domain (residues 35-144), of hCEA share regions of high homology.

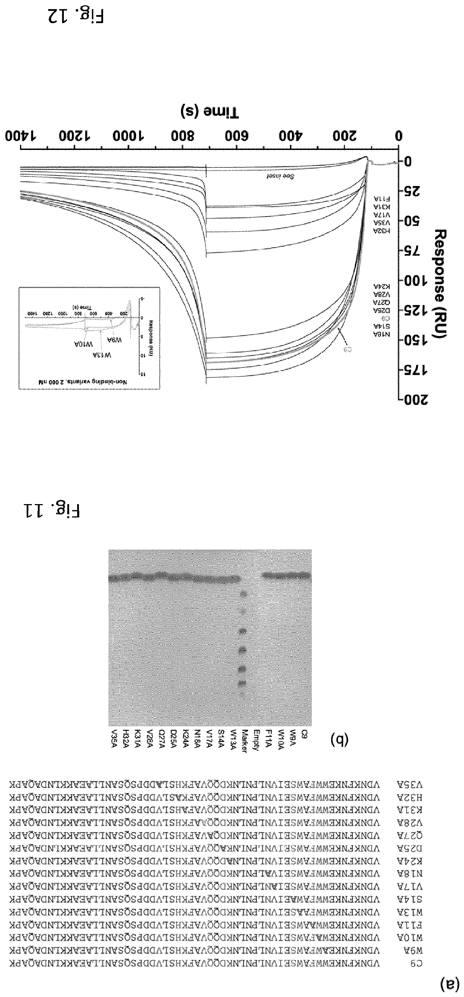

Figure 11: Design and production of 14 individual alanine mutated variants of the affibody clone C9. (a) The amino acid sequences of individual alanine mutated variants of clone C9 are listed, with the parental C9 sequence included as a reference (top row). Each of the 14 affibody positions (9, 10, 11, 13, 14, 17, 18, 24, 25, 27, 28, 31, 32 and 35, all highlighted), were mutated to alanine and included in the combinatorial randomization to construct the libraries. (b) Wild type C9 clone and the 14 alanine mutants were produced as affibody-His₆ fusion proteins in *E. coli* and purified from cell lysates under native conditions via immobilized metal ion chromatography (IMAC), The purity of each produced construct was assessed by SDS-PAGE analysis.
Figure 12: Substitution of specific tryptophan residues in C9 abrogates binding to hCEA. The binding capacity of 14 alanine substituted C9 variants was compared to parental C9 (C9 WT,). The binding of each monovalent affibody-His₆ protein to immobilized hCEA is presented. A concentration of 200 nM was used for all variants. Binding of the variants N18A, S14A, D25A, Q27A, V28A and K24A was unaffected compared with parent C9, whereas variants H32A, V35A, V17A, K31A and F11A displayed markedly reduced binding responses. Interestingly, the three variants W9A, W10A and W13A did not bind to hCEA. A second analysis confirmed this total loss of affinity following each of the W9A, W10A and W13A variants, using a high concentration of 2000 nM (inset).

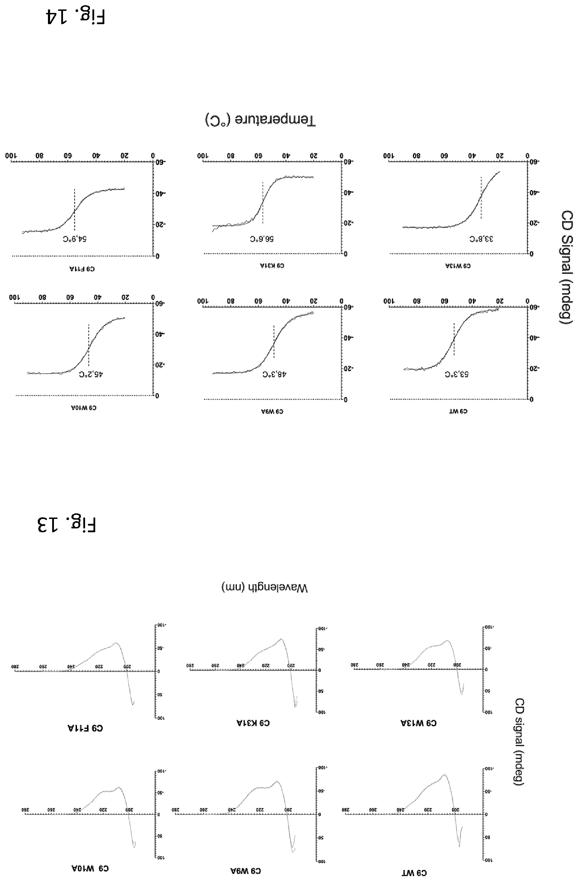

Fig 13: Secondary structure analysis of alanine substituted variants of clone C9 using circular dichroism spectroscopy. The five variants of C9 that were most affected for their binding to hCEA were analyzed by circular dichroism spectroscopy to monitor their secondary structure contents, compared to the parental C9 affibody. Proteins were analyzed as affibody-His₆ fusion proteins. The results showed that all six analyzed proteins had comparable secondary structure contents (mdeg levels), and characteristic minima at 212 and 221 nm for proteins with a large relative content of alpha helices.
Figure 14: Circular dichroism thermal melting point analyses indicate that single alanine replacements significantly affect the overall stability of different C9 variants. The five alanine substituted variants of C9 that were most affected in their binding to CEA were also analyzed by circular dichroism spectroscopy to monitor their thermal melting points, compared to the parental C9 affibody. Proteins were analyzed as affibody-His₆ fusion proteins. The two variants K31A and F11A had higher Tₘ values compared to parental C9, while W9A and W10A had reduced Tm values, reflecting lower overall stability. The clone W13A was most affected by the alanine substitution, with a decreased Tₘ value of 19.5°C compared to parental C9, although still over 33°C.

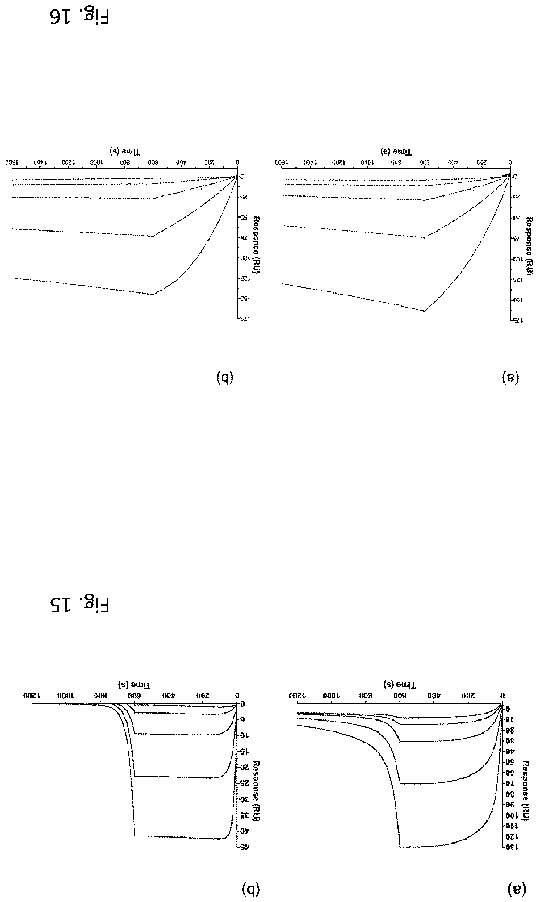

Figure 15: Binding of C9 to hCEA is independent of glycosylation. Untreated (a) or PNGase F-treated (b) biotinylated hCEA was captured on separate streptavidin containing sensor chip surfaces. Injection of a concentration series of C9-His₆ protein over both surfaces demonstrated that C9 recognized both forms of CEA, and thus indicating that the epitope on CEA does not involve N-linked carbohydrates. The curve fitting was improved for PNGase F-treated samples indicating a more homogenous ligand on the chip.
Figure 16: The binding of scFv MFE-23 to CEA is not affected by PNGase F treatment. A series of concentrations of the MFE-23 scFv ranging 1-200 nM was injected over either un-treated (a) or PNGase F-treated hCEA and the responses recorded. The analysis showed that the MFE-23 binding profiles were very similar to the differently treated hCEA samples, and thus indicate that the MFE-23 scFv epitope on hCEA does not involve N-linked carbohydrates.

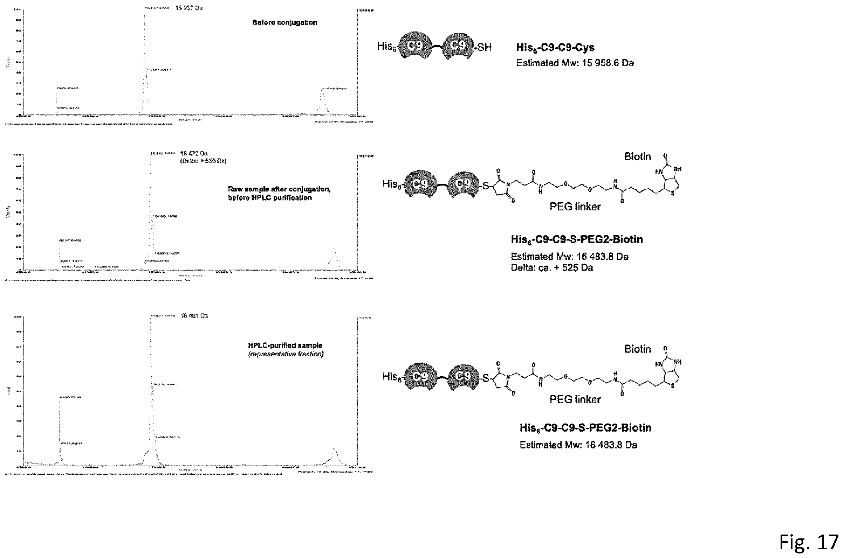

Figure 17: High performance liquid chromatography and mass-spectrometry analyses of the His₆-C9-C9-PEG-Biotin conjugate used in the immunohistochemistry assays reveal high purity. High performance liquid chromatography (HPLC) was used to analyze and purify the His₆-C9-C9-PEG-Biotin conjugates used in the immunohistochemistry (IHC) experiments. Top panel: Crude His₆-C9-C9-Cys protein after IMAC purification. Middle panel: Raw sample after conjugation between His₆-C9-C9-Cys and the maleimide-PEG2-Biotin conjugate. Bottom panel: Post-purification analysis of HPLC-purified His₆-C9-C9-PEG-Biotin conjugate. These results showed that the conjugation was efficient, giving a mass increase of ca. 535 Da (close to the expected value), providing highly pure and homogenous product for immunohistochemistry. Molecular masses were determined with MALDI (TOF/TOF) on a Sciex instrument of medium accuracy and resolution.

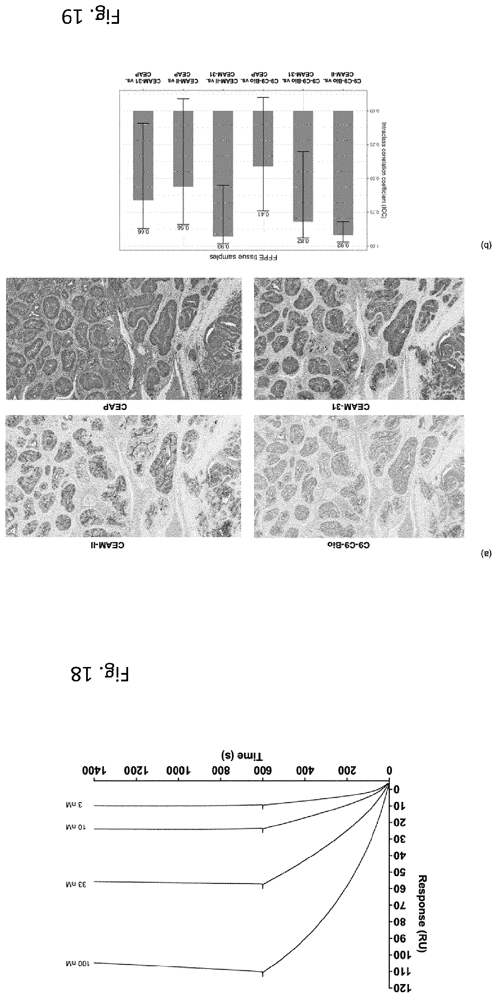

Figure 18: Biosensor analysis of the His₆-C9-C9-PEG-Biotin conjugate. The ability of the purified His₆-C9-C9-PEG-Biotin conjugate to bind to hCEA was assessed using surface plasmon resonance. A concentration series (3-100 nM) was injected over a hCEA-coated sensor chip surface. The apparent affinity for hCEA was calculated to ca. 1.8 nM, using a 1:1 Langmuir binding model. This value of the apparent affinity was well in line with values obtained for unconjugated C9-C9-His₆ protein, indicating that the conjugation and HPLC steps had not had any negative effect on the hCEA-binding functionality.
Figure 19: The homodimeric C9-C9 affibody allows for unambiguous detection of hCEA in FFPE samples. (a) Representative photomicrograph of hCEA detection with the dimeric C9-C9-Bio reagent and the clinical-grade monoclonal and polyclonal antibodies. (b) Intraclass correlation coefficients (ICC) as measurement of agreement in CEA detection scoring between the dimeric C9-C9-Bio reagent and the monoclonal and polyclonal antibodies.

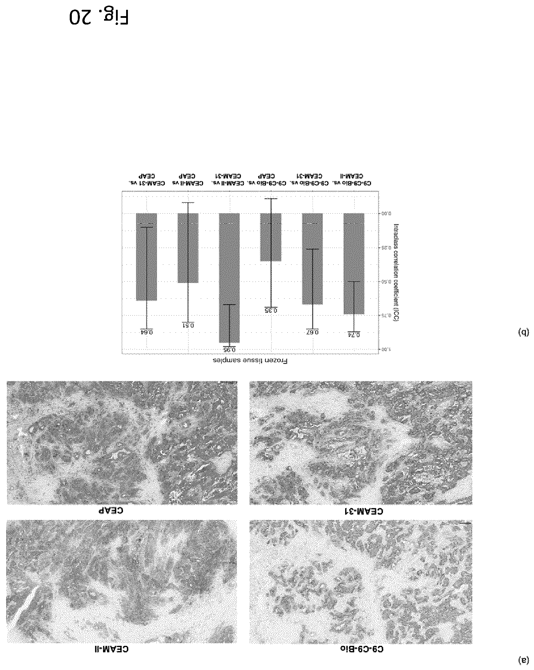

Figure 20: CEA detection in tissue sections using the homodimeric C9-C9 affibody. (a) Representative photomicrograph of hCEA detection with the dimeric C9-C9-Bio reagent and the clinical-grade monoclonal and polyclonal antibodies. (b) Intraclass correlation coefficients (ICC) as measurement of agreement in CEA detection scoring between the dimeric C9-C9-Bio reagent and the monoclonal and polyclonal antibodies.

### DETAILED DESCRIPTION

According to a first aspect, there is provided CEA-binding polypeptide comprising at least one alpha helix and a CEA-binding motif, wherein the CEA-binding motif is located on the at least one alpha helix and comprises the amino acid sequence XₐX_{b}X_{c}X_{d}Xₑ (SEQ ID NO:1), wherein
Xₐ is For W;
X_{b} is W;
X_{c}, and X_{d} are individually selected from any amino acid; and
Xₑ is W or Y.

According to a second aspect, there is provided a fusion protein or conjugate comprising at least:
- a first part comprising the CEA-binding polypeptide as described hereinbefore; and
- a second part comprising at least one additional CEA-binding polypeptide as described hereinbefore, a therapeutic agent, a labelling agent, a half-life-extending region, and/or a second target-binding polypeptide.

According to a third aspect, there is provided a conjugate comprising at least:
- a first part comprising the CEA-binding as described hereinbefore or the fusion protein or conjugate as described hereinbefore; and
- a nanoparticle.

According to a fourth aspect, there is provided the CEA-binding polypeptide, fusion protein or conjugate as described hereinbefore for use in a method of treatment of a disorder, preferably wherein the disorder is cancer.

According to a fifth aspect, there is provided the use of the CEA-binding polypeptide, or the fusion protein or conjugate as described hereinbefore for the in vitro diagnosis of the presence and/or level of CEA in a sample.

### Examples

### Materials and methods

### Affibody libraries

The affibody phage display libraries used for selections within this study have been constructed earlier [5], and were based on *lac* promoter controlled phagemids containing an Omp A signal peptide. These libraries were designed for phage display of encoded affibody library members as in-frame fusions to an albumin binding domain (ABD), an amber stop codon and either a full-length (Lib-1) or a truncated version (aa 249-406) (Lib-2) of M13 phage protein 3. Lib-1 contained also a trypsin sensitive amino acid sequence defined by SEQ ID NO:57 [6] in front of p3 to facilitate proteolytic elution of phage during selections.

In both libraries, 14 positions (pos. 9, 10, 11, 13, 14, 17, 18, 24, 25, 27, 28, 31, 32 and 35) in helices 1 and 2 of the affibody scaffold were randomized using trinucleotide codon technology. Codons for all 20 amino acids except Cys and Pro were allowed at an even distribution in all positions except 31, where a more restricted mixture of 60% Ile and 10% each of His, Tyr, Lys and Asp, was allowed. The estimated complexities of Lib-1 and Lib-2 were 4.7 x 10⁹ and 3.0 x 10¹⁰, respectively.

### Phage stock preparations

Phage stocks for first round selections were produced from *E. coli* ER2738 library cell stocks using standard procedures as previously described [5,7] using either KM13 helper phage (Creative Biolabs, New York, USA) for Lib-1 or M13KO7 helper phage (New England Biolabs, Massachusetts, USA) for Lib-2, where KM13 helper phage encode a trypsin sensitive version of p3. *E. coli* XL-1 Blue cells were used for phage stock production in successive cycles and for phage-ELISA experiments.

### Selection, phage-ELISA screening and DNA sequencing

Selections were performed essentially as previously described [5,7].Briefly, the affibody phage libraries were subjected to four rounds of panning to biotinylated CEACAM-5 (CEA)-Avitag-His₆ protein (CE5-H82EO, Aero Biosystems, Delaware, USA) coupled to streptavidin-coated paramagnetic beads (M280-SA, ThermoFisher, Massachusetts, USA). In successive cycles, the target protein concentration was decreased from 200 nM in cycle 1 to 25 nM in cycle 4, and the number of washes increased from one (cycle 1) to ten (cycle 4).

For both libraries, phage-target protein complexes were eluted from the beads using either low pH (Hac, pH 2.8) or trypsin (0.25 mg/ml, 30 min) in parallel tracks. After the fourth cycle, individual clones were screened for target binding using phage-ELISA with individually prepared monoclonal phage stocks. Microtiter plates were coated with human serum albumin (HAS) (positive control, binding to an ABD domain present on all affibody-displaying phage particles), CEA, streptavidin as a negative control and BSA as an additional negative control. A mouse monoclonal anti-M13-horseradish peroxidase (HRP) conjugate (Merck, Steinheim, Germany) was used together with a 3,3', 5,5' tetramethylbenzidine (TMB) substrate for detection. Reactions were stopped using 2 M H₂SO₄ and plates were read at 450 nm using a plate-reader (Clariostar, BMG Ortenberg, Germany). The affibody gene inserts in twenty-four phage-ELISA positive clones per track were sent for DNA sequencing (Microsynth Seqlab, Göttingen, Germany).

### Cloning, protein expression and purification

Affibody gene inserts of selected clones were amplified by PCR and inserted via In-Fusion cloning from Takara (Shiga, Japan) into an in-house T7 promoter-based expression vector for intracellular production of His₆-affibody-ABD fusion proteins in *E*. *coli* BL21(DE3) cells. Protein production followed standard procedures including the induction of overnight cultures grown at 37°C with 0.5 mM IPTG. Proteins were purified from either lysed or sonicated samples by immobilized metal ion chromatography (IMAC) under denaturing or non-denaturing conditions, including elution with 300 mM imidazole. Based on the anti-CEA affibody clones C9 and C11, second-generation constructs including C9-His₆, C9-C9-His₆, C11-C11-His₆, His₆-C9-Cys, His₆-C9-C9-Cys and His₆-C11-C11-Cys were assembled via In-Fusion cloning and produced using similar protocols.

Further, a set of 14 individual C9 variants in which the randomized positions 9, 10, 11, 13, 14, 17, 18, 24, 25, 27, 28, 31, 32 and 35 were mutated to alanine, were constructed via PCR mutagenesis in the format C9_{Ala mut}-His₆. Sequences of all constructs were verified by DNA sequencing (Eurofins, Ebersberg, Germany) and proteins produced and purified as described above. Circular dichroism spectroscopy analyses were performed on an Applied Photophysics instrument (Leatherhead, United Kingdom) on the wild type C9 affibody and all alanine variants where binding was strongly affected (W9A, W10A, F11A, W13A) to investigate the secondary structure contents and thermal stability.

### MFE-23 scFv cloning, expression and purification

The sequence of the anti-CEA scFv fragment MFE-23 was obtained from the Protein Structure Data Base (PDB) entry 1QOK.pdb. An expression construct in the vector pET22b(+) including a T7 promoter, PelB signal peptide, C-terminal His₆ tag was obtained from GenScript Biotech BV (Netherlands). *E. coli* BL21(DE3) was used for expression and the protein was purified via IMAC from periplasmic preparations obtained via a standard osmotic shock procedure [8].

### Biosensor binding studies

Surfaces with immobilized CEA (Acro BioSystems, human CEACAM5 His-tag, cat. CE5-H5226, 2850 RU) and HAS (Sigma, 3400 RU) were prepared via standard amine coupling on a Biacore 3000 instrument (Cytiva) CM5 chip and used for the initial binding studies. Samples of His₆-affibody-ABD fusions were injected at 200 nM for an initial characterization. Dilution series (1-2000 nM, 2-fold dilutions) of His₆-C9-ABD and His₆-C11-ABD were injected to estimate binding affinities. Monomeric C9-His₆ and C11-His₆ as well as head-to-tail dimers His₆-C9-C9 and His₆-C11-C11 were injected at 200 nM to investigate avidity effects from dimerization.

For binding epitope analyses C9-C9-His₆ was immobilized (800 RU) via amine coupling on a series S CM5 chip on a Biacore T200 instrument (Cytiva). Injection of CEA (50 nM; same as above) alone was compared with injections of CEA pre-mixed (1 h, RT) with 10-fold molar excess of C9-C9-His₆, C11-C11-His₆ or scFv MFE-23. Affinity of MFE-23 scFv was measured by injecting a dilution series (1-100 nM, 3-fold dilutions) over a surface with freshly immobilized CEA (2500 RU). Ala-mutants of affibody C9 (affibody-His₆) were injected (200 nM) over 3000 RU of biotinylated CEA (Aero Biosystems, CE5-H82E0) captured on 2600 RU of streptavidin (Thermo Scientific, #21122) immobilized via amine coupling on a Biacore T200 series S CM5 chip. Variants with no apparent binding were injected also at 2000 nM. Binding measurements to deglycosylated CEA were performed on a Biacore T200 using differently treated samples of biotinylated CEA (ca. 3000 RU) immobilized in separate flow cells of a CM5 chip with amine coupled streptavidin (ca. 2600 RU) followed by injection of affibody (C9-His₆; 2.5-200 nM, 3-fold dilutions).

Samples of untreated CEA, PNGase F- and heat-treated CEA and only heat-treated CEA were evaluated in parallel. PNGase-F (PNGase F glycan cleavage kit, Gibco, Cat. A39245) treatment was performed according to the manufacturer's recommendations (1 h, 50°C) prior to capture on streptavidin. For validation, a similar experiment was performed on a new chip using CEA that was deglycosylated at 37°C ON with four-fold more PNGase-F (data not shown). All experiments were performed at 25°C and 30 µl/min flow rate using PBST pH 7.4 as running buffer and 10 mM HCl for regeneration. Curve fitting was done using a Langmuir 1:1 binding model in the software BIAEval 3.2.1.

### Biotinylation

His₆-C9-C9-Cys protein was biotinylated at the C-terminal 24ysteine using a maleimide-PEG2-biotin conjugate (Thermo Fisher, Massachusetts, USA), according to the instructions from the supplier. After conjugation, the sample was purified using HPLC on a C18 column (Agilent, Santa Clara, USA) and analyzed by MALDI TOF/TOF mass spectrometry (Sciex, Massachusetts, USA).

### Handling of clinical human pancreatic cancer samples

Human pancreatic cancer samples were collected from surgical resection specimens from (n = 7) patients who underwent surgery at the Karolinska University Hospital between March 2021 and March 2022. A piece of each tumor was sampled by a specialized pancreatic pathologist and thereafter divided in two parts. One part was fixed in formalin and embedded in paraffin, and the second part snap frozen in liquid nitrogen and stored at -80 °C until use. The series consisted of n=6 pancreatic ductal adenocarcinomas (PDAC) and n=1 pancreatic neuroendocrine tumor (PanNET).

### Affibody and immunohistochemical (IHC) staining

Formalin-fixed paraffin-embedded (FFPE) and frozen samples were cut into 5 µm and 4 µm thick sections , respectively. The following stainings were performed using an automated immunostainer (Leica BOND III or Ventana Benchmark Ultra), and in-house optimized protocols with anti-human CEA head-to-tail dimeric C9-C9-Bio affibody, mouse monoclonal CEA-II-7 (Dako, Agilent, Santa Clara, USA), mouse monoclonal CEA31 (Ventana, Roche Diagnostics, Indianapolis, USA) and rabbit polyclonal CEA (Dako, Agilent). Information on the antibodies and staining protocols is summarized in Table 3.

**Table 3: Overview of the immunohistochemistry systems used in this study**

| | **C9-C9-Bio** | **mAb II-7** | **mAb CEA31** | **Polyclonal Ab** |
|---|---|---|---|---|
| Source | This study | Dako (Agilent) | Ventana (Roche) | Dako (Agilent) |
| Product code | N/A | M7072 | 06433316001 | A0115 |
| Used concentration | 3.2 µg/mL | 0.518 µg/mL (diluted 1:400) | 0.46 µg/mL (ready-to-use) | 0.5 µg/mL (diluted 1:4000) |
| Platform | Bond-III Leica | Bond-III Leica | ultraBenchmark Ventana | ultraBenchmark Ventana |
| Detection | Bond Intense R | DAB Refine | DAB ultra View | DAB ultra View |

Details on the staining protocols for the C9-C9-affibody (FFPE and frozen tissues) are presented in Table 4 hereinbelow. After staining, the slides were digitalized using a Hamamatsu NanoZoomer scanner at 20X magnification. Two specialized pancreatic pathologists evaluated the digital slides and scored by consensus all the stainings in the following cell and tissue compartments: apical membrane, cytoplasm, and budding-like nests in the cancer cells, stromal cells, and normal epithelium. Scores were based on the percentage of stained cells and for diffuse scores (> 95%) intensity was also included as a weight (moderate: 1.15, and strong: 1.30), multiplied by the extent of positive immunoreactivity. Data and statistical analyses were performed using R (v 4.2.1). The level of agreement between stainings was calculated in a pairwise manner based on the intraclass correlation coefficient (ICC) using the irr package ("twoway" model, "agreement" type, "single" unit) and Bland-Altman plots depicting the bias and 95% upper and lower limits of agreement. The ICCs were interpreted as poor: <0.5, good: 0.5-0.75, moderate: 0.75-0.9, and excellent: >0.90.

**Table 4: Details of staining protocols used for the C9-C9-Biotin reagent**

| | **Affibody reagent Frozen sections** | **Affibody reagent FFPE sections** |
|---|---|---|
| 1 | Bond wash [0.3 min] | Dewax (deparaffinization) [0.3 min] |
| 2 | 1% BSA-PBS [15 min] | HIER (pretreatment) with EDTA pH 9 [20 min] |
| 3 | Peroxide block [5 min] | Bond wash [0,3 min] |
| 4 | Streptavidin block [15 min] | 1% BSA-PBS [15 min] |
| 5 | Biotin block [15 min] | Peroxide block [5 min] |
| 6 | Marker (His₆-C9-C9-Cys-PEG-Biotin) [45 min] | Streptavidin block [15 min] |
| 7 | Streptavidin-HRP (Bond Intense R) [8 min] | Biotin block [15 min] |
| 8 | Mixed DAB [5 min] | Marker (His₆-C9-C9-Cys-PEG-Biotin) [45 min] |
| 9 | Hematoxylin [5 min] | Streptavidin-HRP (Bond Intense R) [8 min] |
| 10 | - | Mixed DAB [5 min] |
| 11 | - | Hematoxylin [5 min] |
| **Material for biotin-based staining (affibody reagent):** | | |
| Bond Intense R Detection: DS9263 | | |
| Diluent: Bond primary diluent: AR9352 | | |
| Biotin solution: 30011 Vector Laboratories | | |
| Streptavidin solution: 30088 Vector Laboratories | | |

### Results

### Identification, characterization and validation of CEA-specific affibodies

In order to identify small affibody affinity proteins with high specificity and affinity to the human carcinoembryonic antigen-related cell adhesion molecule 5 (human CEACAM-5, CEA), two combinatorial phagemid-based affibody phage libraries (Lib-1 and Lib-2) were used for *in vitro* selection campaigns towards a recombinant biotinylated CEA target protein produced in human HEK293 cells and containing all seven Ig-like domains (corresponding to aa 35-685) (Figure 1).

The two libraries differed in regard to the variant of phage coat protein 3 used as gene fusion partner in the phagemids, and the helper phages used for their preparation (Figure 2). During selections, streptavidin bead-captured phage-target complexes were eluted in parallel using either low pH (pH 2.6) or trypsin cleavage, to potentially broaden the diversity of the output in regard to the characteristics of affibody-target interactions. After four cycles of selections, a total of 94 output clones were randomly picked from all tracks and analyzed for hCEA binding using a microtiter-scale monoclonal phage-ELISA assay. The proteins bovine serum albumin (BSA) and streptavidin were used as negative controls, and human serum albumin (HSA) as positive control, the latter expected to be recognized by all affibody-displaying phage particles via an albumin binding domain (ABD) expressed in fusion with the affibody library members, and thus also displayed on the phage surface. Phage-ELISA analyses (Figure 3) showed that a majority of the 192 analyzed clones displayed clear binding to both hCEA and HSA, with very low background binding to the negative controls. A total of 96 phage-ELISA positive clones from both libraries, and from both acid and trypsin elution tracks, were selected for further analyses.

The affibody gene inserts of the 96 clones were DNA sequenced, allowing identification of several sequence clusters by phylogenetic analyses (Figure 4). Some clones had been highly enriched during selections and were represented several times, including clones B6 (14x), from Lib-1 and C9 (17x) and F7 (8x) from Lib-2, whereas other clones were represented by single hits. Some clones were found in outputs from both acid and trypsin elution tracks (Figure 5). Interestingly, 13 clones comprised tryptophan residues at both positions 9 and 10 in helix 1, and four additional clones had a phenylalanine and a tryptophane residue at positions 9 and 10, respectively. Furthermore, two clones (B6 and H11) comprised either of WW or FW combinations at positions 27 and 28 in helix 2. Altogether, 19 unique clones were identified. All unique variants were reformatted as His₆-affibody-ABD fusion constructs (Figure 1c-i) and produced as soluble proteins in *E. coli.*

The 19 unique variants were all analyzed for binding to hCEA using surface plasmon resonance (SPR) biosensor technology. Separate injections of the His₆-affibody-ABD fusions at a 200 nM concentration over a sensor chip surface containing immobilized hCEA revealed a wide distribution of response signal strengths and binding kinetics (Figure 6a). Binding responses of the fusion proteins to an HSA-containing sensor chip surface, via the included ABD moiety, indicated that the injected concentrations were similar and that none of the fusion proteins formed multimers or aggregates (Figure 6b). Two of the clones, C9 and C11, displayed the highest binding responses and slowest off-rate kinetics, and were therefore selected for further characterization. Monovalent 1:1 binding affinities (K_{D}) of both these clones to hCEA were determined from injections of serial dilutions to ca. 200 nM, albeit with somewhat different binding profiles where the C9 variant showed slower off-rate kinetics (Figure 7).

To investigate if the apparent affinity to hCEA could be increased via avidity effects, head-to-tail dimer fusions were constructed (Figure 1c-ii) and evaluated for their binding affinities to sensor chip-immobilized hCEA. The results demonstrated that both dimer constructs C9-C9-His₆ and C11-C11-His₆, in which the two affibody moieties were connected via a 15 aa flexible linker, defined by (SEQ ID NO:59)₃, displayed significantly higher overall binding responses. Furthermore, both these head-to-tail dimer constructs displayed markedly slower off-rate kinetics compared to their monomeric counterparts, indicating a clear avidity effect and thus that both affibody moieties were functional when produced in this dimeric format (Figure 8).

Using a sensor chip surface containing immobilized C9-C9-His₆ protein, a series of epitope binning experiments were performed. First, injections of either free hCEA or hCEA pre-mixed with a 10-fold molar excess of either C11-C11-His₆ or C9-C9-His₆ proteins over the sensor chip surface, revealed that pre-mixing of hCEA with C11-C11-His₆ abolished the binding signal seen for free hCEA to immobilized C9-C9-His₆, indicating the presence of overlapping epitopes on hCEA for C9 and C11 (Figure 9). As expected, a similar blocking was seen after pre-mixing hCEA with C9-C9-His₆ (self-blocking). Further, the binding profile obtained from injection of free hCEA over C9-C9-His₆ resembled the 1:1 binding profile obtained in the reversed format using a monovalent C9 fusion protein (Figure 8). This indicates that hCEA, although composed by multiple domains with high sequence homology (Figure 10), contains only one single epitope for the C9 and C11 affibodies, as otherwise slower off-rate kinetics from di- or multi-site binding would have been expected.

To further characterize the binding of the affibodies to hCEA, a previously described anti-hCEA scFv denoted MFE-23 was also used in binding competition experiments. This scFv has been previously proposed to bind to a less glycosylated pocket of hCEA, localised between the first two N-terminal domains N1 and A1 [3] (Figure 1b). Experimental support for the presence of an epitope for MFE-23 scFv within the N1-A1 region of CEA was also provided from binding studies using a recombinant N1-A1 protein [9]. In the present study, the functionality of an in-house produced MFE-23 was first assessed in a direct binding experiment to immobilized hCEA, where it was found to bind with an affinity of ca. 15 nM (Figure 9, inset). Interestingly, pre-mixing hCEA with a 10-fold molar excess of MFE-23 scFv abolished binding of hCEA to C9-C9-His₆ (Figure 9). Taken together, these experiments indicate that MFE-23 scFv as well as affibodies C9 and C11 all bind to overlapping epitopes within the two N-terminal domains of hCEA.

### Alanine scanning studies of affibody clone C9 reveals the importance of individual residues for affinity to hCEA

To investigate the relative importance of individual amino acid residues on the affibody binding surface for recognition of hCEA, a set of 14 individual alanine mutated variants corresponding to the positions randomized during the library construction were cloned, expressed, purified as *C*9*_{Ala mutant}-*His₆ constructs (Figures 1c-iii and Figure 11), and subjected to hCEA binding studies with wildtype C9 affibody as a reference. The results demonstrated that alanine substitutions at positions 14, 18, 24, 25, 27 and 28 did not significantly affect binding, while substitutions at positions 11, 17, 31, 32 and 35 slightly decreased the binding affinity. However, alanine substitutions at positions 9, 10 and 13, all occupied by tryptophan residues in C9, abrogated binding to hCEA (Figure 12). Circular dichroism spectroscopy analyses of these alanine-substituted variants did not indicate any substantial differences in their secondary structure contents compared to the wild type C9 affibody (Figure 13).

Further, thermal melting point analyses were performed on the affibody variants in which each of the three tryptophan residues W9, W10 and W13 and residues F11 and K31 were mutated to alanine. While the W9A and W10A substitutions reduced the thermal stability by 5-8°C, a substitution at position 13 to alanine resulted in a decreased thermal stability of close to 20°C (Figure 14). Interestingly, alanine substitutions at positions F11 and K31 resulted in slightly increased thermal stabilities. Taken together, these results indicate that the almost complete loss of hCEA binding observed for two of the three tryptophan mutants (W9A and W10A) did not correlate with a correspondingly large loss of secondary structure or thermal stability, and that instead effects from alterations in their binding interfaces to hCEA most likely caused the dramatic loss of binding. However, for the third tryptophan mutant (W13A) it cannot be excluded that the reduced thermal stability affected its binding functionality, although the binding analysis was performed at 25°C which is significantly below its melting temperature of 33.8°C.

### The affibody C9 binds with high affinity to a non-glycosylated epitope on hCEA

The importance of the three tryptophan residues for recognition of hCEA by C9 prompted us to investigate the influence of glycosylation for these interactions. Tryptophans have been previously reported to be heavily overrepresented in proteins that recognize carbohydrate structures [10] and since hCEA has been predicted to contain at least 28 N-linked glycosylations (Uniprot ID: P06731), the likelihood for an important role of hCEA carbohydrates in these interactions could be significant. To this end, streptavidin-coated sensorchip surfaces were employed for binding studies between the head-to-tail dimeric C9-C9-His₆ affibody and biotinylated hCEA that was either untreated or treated with PNGase F, an enzyme that effectively removes N-linked oligosaccharides from proteins. Interestingly, our results demonstrated that binding of C9 to hCEA was not significantly affected by the removal of all carbohydrates, indicating that the C9/hCEA interactions involve a carbohydrate-free region in hCEA (Figure 15). Interestingly, parallel experiments demonstrated that binding of the MFE-23 scFv to CEA was also unaffected by PNGase F-mediated deglycosylation (Figure 16).

Taken together, the experimental data indicates i) a binding site within the N-terminal domains of hCEA that ii) is present only in a single copy in the full-length hCEA protein, iii) does not involve carbohydrates and iv) depends on all three tryptophans or other large hydrophobic amino acids. Interestingly, in contrast to the A and B domains of hCEA, the N-terminal N1 domain does not share any large homologies to other parts of the sequence, which is in line with the data indicating a single binding site in hCEA (Figure 10).

### Unambiguous detection of hCEA in pancreatic cancer samples using dimeric C9-C9 affibody

The high affinity binding to hCEA observed for the dimeric C9-C9 construct (Figure 8) prompted us to investigate its potential use for detection of hCEA in complex biological samples. To this end, a homodimeric His₆-C9-C9-Cys construct (Figure 1c-iii) was assembled to assess the immunohistochemical capacity of this construct to detect hCEA in human pancreatic cancer specimens. The C-terminal cysteine residue of the construct was recruited for site-specific biotinylation using maleimide-PEG-biotin (Figures 1c-iv and Figure 17) for detection via a streptavidin-HRP conjugate. Our SPR analyses demonstrated that the His₆-C9-C9-Cys-PEG-Biotin conjugate (C9-C9-Bio) bound to hCEA with an affinity of approximately 2 nM (Figure 18), well in line with our initial binding experiment results using the unconjugated head-to-tail dimeric C9-C9-His₆ protein.

Depending on the affinity reagent used and the sample type (frozen or formalin-fixed paraffin-embedded, FFPE cryotome tissue sections), different instrumentation and protocols were applied (Tables 3 and 4). The performance of the C9-C9-Bio conjugate in adequate detection of hCEA within tissue samples was compared to both monoclonal anti-CEA antibodies II-7 from Dako/Agilent, and CEA-31 from Ventana/Roche (Table 3), as well as a polyclonal reagent (Dako/Agilent). All of the comparators are used routinely in clinical diagnostics. Two pancreas pathologists assessed independently the different stains first at a qualitative level. The overall staining pattern was highly consistent between C9-C9-Bio and the two monoclonal antibodies regarding the extent and the spatial location of the stained cancer cells, as well as negative reactivity in non-malignant cells (stromal, inflammatory, and normal epithelium), and the absence of background staining.

It should however be noted that the C9-C9-Bio reagent produced a highly accurate and defined hCEA detection with apical membrane accentuation, compared to the monoclonal antibodies (Figures 19A and 20A). The staining pattern of C9-C9-Bio was very similar to that of CEA-II, while CEA-31 yielded a more saturated stain with cytoplasmic reactivity, more in line with the results usually obtained with the polyclonal antibody reagent (Figures 19A and 20A). The latter polyclonal product produced a poorly specific staining with reactivity in stromal cells and background.

The stainings were further evaluated quantitatively in a pairwise manner based on the pathologists' scores. On FFPE tissues C9-C9-Bio displayed an excellent and moderate intraclass correlation coefficient (ICC) agreement with the monoclonal antibodies CEA-II and CEA-31, respectively. As expected, its agreement was poor with the staining obtained with the polyclonal reagent, showing a diffuse staining (Figure 19B). These results were highly consistent with the qualitative assessment. The agreement of ICCs between C9-C9-Bio and the monoclonal antibodies were lower but in the good range for frozen tissues (Figure 20B). Similarly, Bland-Altman analysis demonstrated that the agreement in CEA detection was highest between C9-C9-Bio and monoclonal CEA-II, followed by monoclonal CEA-31 (bias = -7 and -19 respectively), and that the performance of C9-B9-Bio was superior in FFPE compared to frozen tissues (Figure 20b).

### Discussion

As demonstrated in the Example section and through extensive evaluation of CEA-specific affinity proteins, a CEA-binding motif enabling high-affinity interaction with CEA was identified. Through an extensive phage display screening process, the CEA-binding motif was selected based on its ability to provide strong and specific binding to CEA. The identified sequence motifs, particularly those rich in tryptophan (W) and tyrosine (Y), were found to play critical roles in the high-affinity binding observed in these studies. Replacement of these residues with alternative amino acids, such as alanine, resulted in a significant reduction or complete loss of binding ability. This underscores the importance of these conserved residues in forming stable interactions with CEA.

The results from the alanine scanning, as described hereinbefore, also indicate that several residues in the CEA-binding polypeptide may be replaced without compromising the binding to CEA (as long as the CEA-binding motif remains intact). In some positions the accepted replacement is not conservative i.e. alanine is separated with regard to properties. It is expected that chemically and structurally conservative amino acid replacements can be done without essentially affecting affinity to CEA also in positions where an effect on binding is seen after alanine replacement. This prediction is supported by a comparison of the two strongest binders C9 and C11, showing similar affinity despite differing in 11 of the 14 varied amino acids, while retaining the important motif of three hydrophobic amino acids.

Furthermore, affibody constructs containing the identified CEA-binding motif demonstrated robust and specific binding in various experimental conditions, including frozen and formalin-fixed, paraffin-embedded (FFPE) tissue samples. The high specificity and low background staining achieved with these constructs highlight their potential as effective affinity agents for immunohistochemical (IHC) applications.

Glycosylation-independent binding was confirmed by experiments in which PNGase F-treated CEA samples retained strong binding to the affibody constructs, despite the removal of N-linked carbohydrates. This finding is significant given the highly glycosylated nature of CEA and the fact that many affinity reagents suffer from cross-reactivity due to carbohydrate interactions. The Alanine scan confirmed that tryptophans in key positions were essential for binding of C9, and competitive binding studies with the MFE-23 scFv indicated that the identified motif may bind to a specific epitope within the N1 domain of CEA, and does not include carbohydrate interactions.

The robust performance of the affibody constructs, particularly in FFPE tissue samples, was noteworthy. FFPE samples undergo extensive processing, including formalin fixation, paraffin embedding, dewaxing, and heat-induced epitope retrieval (HIER), which often compromises the integrity of antigens. The successful staining achieved with the C9-C9-construct under such conditions suggests that the binding epitope of CEA remains stable or is capable of refolding after processing. This contrasts with other affinity reagents that may struggle to retain specificity in such "harsh" conditions.

The performance of the C9-C9-construct in FFPE tissue staining supports its utility in clinical diagnostics. Previous studies on monoclonal antibodies targeting CEA have reported variable results, with some antibodies showing high specificity and sensitivity, while others suffer from cross-reactivity and high background staining. The results obtained using the C9-C9-construct align with those seen for high-performing monoclonal antibodies such as CEA-II but surpass them in terms of reduced background staining and clear apical membrane localization.

The use of the CEA-binding polypeptides of the present disclosure offers several advantages over traditional monoclonal antibodies. The small size of these polypeptides (-16.5 kDa) compared to the much larger antibodies (-150 kDa) allows for deeper tissue penetration and better antigen localization in IHC applications and also for in vivo targeting of therapeutic construct. Additionally, the recombinant or chemical production of affibodies is less complex and costly compared to the production of monoclonal antibodies.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

### References

[1] Nord K, Gunneriusson E, Ringdahl J, Ståhl S, Uhlén M, Nygren PA. Binding proteins selected from combinatorial libraries of an alpha-helical bacterial receptor domain. Nat Biotechnol. 1997 Aug;15(8):772-7. doi: 10.1038/nbt0897-772. PMID: 9255793.
[2] Boehm MK, Corper AL, Wan T, Sohi MK, Sutton BJ, Thornton JD, Keep PA, Chester KA, Begent RH, Perkins SJ. Crystal structure of the anti-(carcinoembryonic antigen) single-chain Fv antibody MFE-23 and a model for antigen binding based on intermolecular contacts. Biochem J. 2000 Mar 1;346 Pt 2(Pt 2):519-28. PMID: 10677374; PMCID: PMC1220881.
[3] Boehm, M.K., Perkins, S.J., 2000. Structural models for carcinoembryonic antigen and its complex with the single-chain Fv antibody molecule MFE23. FEBS Lett 475, 11-16.
[4] www.bioinformatics.nl/cgi-bin/emboss/dotmatcher
[5] Giang, K.A., Boxaspen, T., Diao, Y, Nilvebrant, J., Kosugi-Kanaya, M., Kanaya, M., Krokeide, S.Z., Lehmann, F., Svensson Gelius, S., Malmberg, K.J., Nygren, P.A., 2023a. Affibody-based hBCMA x CD16 dual engagers for NK cell-mediated killing of multiple myeloma cells. N Biotechnol 77, 139-148.
[6] Thomas, W.D., Smith, G.P., 2010. The case for trypsin release of affinity-selected phages. Biotechniques 49, 651-654.
[7] Giang, K.A., Nygren, P.A., Nilvebrant, J., 2023b. Selection of Affibody Affinity Proteins from Phagemid Libraries. Methods Mol Biol 2702, 373-392.
[8] Nossal NG, Heppel LA. The release of enzymes by osmotic shock from Escherichia coli in exponential phase. J Biol Chem. 1966 Jul 10;241(13):3055-62. PMID: 4287907.
[9] Sainz-Pastor, N., Tolner, B., Huhalov, A., Kogelberg, H., Lee, Y.C., Zhu, D., Begent, R.H., Chester, K.A., 2006. Deglycosylation to obtain stable and homogeneous Pichia pastoris-expressed N-A1 domains of carcinoembryonic antigen. Int J Biol Macromol 39, 141-150.
[10] Hudson, K.L., Bartlett, G.J., Diehl, R.C., Agirre, J., Gallagher, T., Kiessling, L.L., Woolfson, D.N., 2015. Carbohydrate-Aromatic Interactions in Proteins. J Am Chem Soc 137, 15152-15160.

## Claims

1. A CEA-binding polypeptide comprising at least one alpha helix and a CEA-binding motif, wherein said CEA-binding motif is located on said at least one alpha helix and comprises the amino acid sequence XₐX_{b}X_{c}X_{d}Xₑ (SEQ ID NO:1), wherein
Xₐ is For W;
X_{b} is W;
X_{c}, and X_{d} are individually selected from any amino acid; and
Xₑ is W or Y.

2. The CEA-binding polypeptide according to claim 1, wherein X_{c} is H, Y, D, V, F, K, G, N, E, or A.

3. The CEA-binding polypeptide according to claim 1 or claim 2, wherein X_{d} is A, or F.

4. The CEA-binding polypeptide according to any one of the preceding claims, wherein said CEA-binding motif comprises the amino acid sequence as defined in any one of SEQ ID NO:2-14, or a corresponding sequence having at least 80% sequence identity with any one of SEQ ID NO:2-14.

5. The CEA-binding polypeptide according to any one of the preceding claims, wherein said CEA-binding polypeptide has a three-helix bundle structure and comprises a first alpha helix, a second alpha helix, and a third alpha helix; said second alpha helix being arranged C-terminally of said first alpha helix; said third alpha helix being arranged C-terminally of said second alpha helix.

6. The CEA-binding polypeptide according to claim 5, wherein said CEA-binding motif is located on said first alpha helix or said second alpha helix of said three-helix bundle structure.

7. The CEA-binding polypeptide according to claim 5 or claim 6 when dependent on claim 4, wherein said CEA-binding motif is located on said first alpha helix and comprises the amino acid sequence defined by any one of SEQ ID NO:2-12, or a corresponding sequence having at least 80% sequence identity with any one of SEQ ID NO:2-12.

8. The CEA-binding polypeptide according to claim 5 or claim 6 when dependent on claim 4, wherein said CEA-binding motif is located on said second alpha helix and comprises the amino acid sequence defined by SEQ ID NO:13 or SEQ ID NO:14, or a corresponding sequence having at least 80% sequence identity with SEQ ID NO:13 or SEQ ID NO:14.

9. The CEA-binding polypeptide according to any one of the preceding claims, wherein said CEA-binding polypeptide comprises the amino acid sequence of any one of SEQ ID NO:15-SEQ ID NO:33 or a corresponding sequence having at least 70% sequence identity, preferably at least 80% sequence identity with any one of SEQ ID NO:15-SEQ ID NO:33.

10. A fusion protein or conjugate comprising at least:
- a first part comprising the CEA-binding polypeptide according to any one of claims 1-9;
- a second part comprising at least one additional CEA-binding polypeptide according to any one of claims 1-9, a therapeutic agent, a labelling agent, a half-life-extending region, and/or a second target-binding polypeptide.

11. The fusion protein or conjugate according to claim 10 when dependent on claim 4 or claim 9, wherein said first part comprises the CEA-binding polypeptide defined in SEQ ID NO:2, SEQ ID NO:3, SEQ ID NO:19, or SEQ ID NO:20, and wherein said second part comprises at least one additional CEA-binding polypeptide as defined in SEQ ID NO:2, SEQ ID NO:3, SEQ ID NO:19, or SEQ ID NO:20.

12. The fusion protein or conjugate according to claim 10 or claim 11, wherein said second part comprises a labelling agent, and wherein said labelling agent is a fluorophore, chromophore, radioactive isotope, or an enzyme.

13. The fusion protein or conjugate according to claim 10 or claim 11, wherein said second part comprises a therapeutic agent, and wherein said therapeutic agent is a cytotoxic agent or a radionuclide.

14. A conjugate comprising at least:
- a first part comprising the CEA-binding polypeptide according to any one of claims 1-9 or the fusion protein or conjugate according to any one of claims 10-13, and
- a nanoparticle.

15. The CEA-binding polypeptide, fusion protein or conjugate according to any one of the preceding claims for use in a method of treatment of a disorder, preferably wherein said disorder is cancer.

16. Use of the CEA-binding polypeptide according to any one of claims 1-9, or the fusion protein or conjugate according to any one of claims 10-14 for the in vitro diagnosis of the presence and/or level of CEA in a sample.
